# EUROPEAN PATENT APPLICATION

(11) **EP 2 108 355 A1**
(43) Date of publication of application: **14.10.2009**
(21) Application number: 08710676.1
(22) Date of filing: 31.01.2008
(51) Int. Cl.: A61J 1/05

(54) **DUPLEX-CHAMBER VESSEL**

(30) Priority: 01.02.2007 JP 2007022557
(71) Applicant: Ajinomoto Co., Inc., Tokyo 104-8315 (JP)
(72) Inventor: MURAMATSU, Yasuhiro, Shizuoka-shi Shizuoka 424-0911 (JP)
(74) Representative: Nicholls, Kathryn Margaret
(86) International application number: PCT/JP2008/051578
(87) International publication number: WO 2008/093806

(57) **Abstract**

A medical bag having a seal separating its inner space into a plurality of partitions for separate sealed storage of medical liquids and aiming for preventing erroneous operation that an infusion is practiced under a non-opened condition without worsening sterilizing performance during the production of the bag.

A weak seal 18 is formed by welding the top and bottom film layers of medical bag 10 along the entire width of the medical bag, so that partitions 20 and 22 are formed at the upper and lower part of the weak seal 18. An outlet port 12 is mounted to the medical bag at a strong seal 14A. The outlet port 12 is extended in the lower chamber 22 to a location adjacent the weak seal 18, so that top end 12A is opened to the lower chamber 22 at a location above the liquid level L. The outlet port 12 is, at its bottom end, formed with a communication hole 26, while the shaded part (valve portion 28) at the inner face of the medical bag is welded to the opposite surface of the outlet port 12, thereby closing the communication hole 26. The welding strength of the valve portion 28 is slightly stronger than that of the weak seal 18.

## Description

### TECHNICAL FIELD

The present invention relates to a multi-chamber container as a medical bag having an inner cavity divided by a weak seal (separable weld or partition wall) into a plurality of partitions for storing therein with medical liquids independently and an outlet port for discharging the medical liquids after mixed from the respective partitions and also relates to a method for discharging medicines.

### BACKGROUND TECHNOLOGY

Known is a multi-chamber container for an infusion, having a medical bag made of soft films and a weak seal as constructed by opposed faces of the films welded at a relatively low temperature in a manner that the bag is divided into a plurality of partitions for storing different medical liquids, respectively. An outlet port as a plastic mold product is arranged at the outer periphery of the bag. The outlet port forms a tubular shape having an inner cavity, which has a first end opened to one of the partitions and a second end fitted with a rubber plug. Prior to giving the medicines to a patient, the medical bag is subjected to a pressing from its outside in a manner the weak seal is opened, which causes the inner chambers of the medical bag to be united, resulting in a mixture of the two different medical liquids. Then, a needle of an infusion set pierces the rubber plug, which makes it possible that the medical liquids from the medical bag is given to the patient. In view of the above, prior to the infusion, a mixing operation of both of the medical liquids by opening the weak seal is essentially necessary. Namely, a piercing of the rubber plug at the outlet port without opening the weak seal causes such an erroneous operation to be occurred that only the medical liquid at the partition located at the partition adjacent the outlet port is given. In order to obviate this problem, an improvement of a medical bag has heretofore proposed wherein it has, in addition to a first weak seal separating the space inside the medical bag into two partitions, a second weak seal located immediately faced with the outlet port. The second weak seal is opened at a value of the pressure, which is the same as or larger than the pressure for opening the first weak seal, so that a sequence of the operation is obtained that the first seal is, at first, opened and, then, the second weak seal is opened, thereby obtaining a discharge of the medical liquids after the mixing. In addition, the second weak seal adjacently faced with the outlet port is formed with a vent hole (communication passage), which allows a gas to be passed while a flow of liquid is blocked (Refer Patent Publication No. 1). This type of multi-chamber container for infusion is usually subjected to a sterilizing by heating at a desired temperature after the completion of a storage of the medical liquids to the respective partitions. Due to the provision of the vent hole in the second weak seal at a location adjacent to the outlet port, the vapor of the medical liquid in the partition on the side adjacent the second weak seal is introduced into the outlet port via the vent hole, so that the vapor is filled not only in the partition but also in the outlet port, thereby executing a sterilizing operation under a wet heat state. If such a provision of the vent hole in the second weak seal were eliminated, an air merely would occupy at the outlet port, so that a dry heat treatment is only attained, resulting in a reduced sterilizing efficiency as compared with that as obtained by the wet type heat treatment. Finally, the vent hole has a small diameter, which is small enough for a blockage of a free flow of the liquid even in a situation that piercing is done under the non-opened condition of the medical bag and, therefore, an occurrence of an erroneous operation, that an infusion is done only under a single liquid state, may be prevented.
Patent Document No.1: Non-Examined PCT Publication No. 2006-507914

### DISCLOSURE OF THE INVENTION

### PROBLEMS TO BE SOLVED BY THE INVENTION

As described above, in the prior art as disclosed in the Patent Document No. 1, at the location adjacently faced with the outlet port, the second weak seal (partition wall) formed with the vent hole is provided for permitting the gas to be freely flown while blocking the flow of liquid. Namely, the vent hole is for keeping a desired sterilization quality, on one hand and, on the other hand, for preventing liquid flow from being occurred even if a piercing of the rubber plug by a infusion set is done under a non-opened condition of the medical bag, i.e., for preventing an erroneous operation from being occurred that an infusion is done under a single liquid state. In order to assure the above function of the vent hole, a strict control of a size of the vent hole as a non-welded portion in the second weak seal is essential, which makes its manufacturing to be difficult in a technological view point. Namely, a design of the vent hole of excessively smaller size may likely generate a problem of reduced sterilization efficiency due to an increased variation in the transmittable amount of the vapor. Contrary to this, an excessively larger size of the vent hole may allow a liquid flow, which causes an erroneous operation to generate that an infusion only of single liquid by way of the vent hole is practiced in case where a piercing is done without opening the medical bag, i.e., without mixing the medical liquids between the partitions. In view of this, it is clear that a desired size control of the vent hole is essential, which is, however, practically very difficult due to the different characteristic between the material properties of films, which makes it to be essential that the vent hole should be designed under a material to material base.

In view of these problems encountered in the prior art, the present invention aims to positively exclude the possibility of occurrence of an erroneous operation that an infusion is done under a non-opened condition without reducing the desired sterilizing capability.

### MEANS FOR SOLVING PROBLEMS

In the present invention, a multi-chamber container is provided comprising: a medical bag made of a flexible film; a separable weld, which connects under a separable manner the opposed surfaces of the medical bag in a manner that the inner space of the medical bag is separated into an upper and a lower partitions for storing medical liquids, respectively; an outlet port having a lower end located outside the medical bag and an upper end opened to the lower partition; a plug connected to the lower end of the outlet port, the plug being for piecing by a infusion set, the upper end of the outlet port opened to the lower partition being located above the level of the liquid in the lower partition under a non-opened condition of the medical bag, the outlet port being formed with a communication hole located at a position adjacent the bottom of the lower partition, and; a member normally closing the communication hole and cooperating with an expanded displacement of the medical bag as initiated when the weld is separated for opening the medical bag in a manner that the opening of the communication hole is succeeded.

The cooperating member may be a portion of the medical bag opposite to the communication hole and said portion is separably welded to the opposed surface of the outlet port in a manner that said portion normally closes the communication hole. A difference in welding strength is provided by a measure such as a higher value of its welding temperature over that for the weak seal (partition wall) between the partitions. As a result, a two-stage opening operation is obtained in a manner that a separation of the weak seal between the partitioned chambers is, first, initiated for mixing of the medical liquids and a separation is, second, generated by a further pressing of the mixed medical bag.

Furthermore, in order to prevent the peeling at the location of the communication hole from being accidentally occurred by a lesser pressing as generated by a handling of the medical bag such as transportation, a suitably measure may be provided such as portions of the medical bag locally separably welded at both sides of the sealed part of the communication hole, non-separable welds extending from the peripheral edge of the medical bag or a holder for holding the outlet port at its top and bottom sides outwardly from the outside of the medical bag.

In addition to the above, a method is provided for discharging medicines from a multi-chamber container to a medical delivery pipe connected to a needle, comprising the steps of:
providing, in the multi-chamber container, a medical bag made of a flexible film, a separable weld, which connects under a separable manner the opposed surfaces of the medical bag in a manner that the inner space of the medical bag is separated into an upper and a lower partitions for storing medical liquids, respectively, an outlet port having a lower end located outside the medical bag and an upper end opened to the lower partition, and; a plug connected to the lower end of the outlet port and
to be pierced by the needle connected to the medical delivery pipe;
locating said upper end of the outlet port in a manner that the upper end is opened to the lower partition at a position above the level of the liquid in the lower partition under a non-opened condition of the medical bag;
providing, in said outlet port, a communication hole located at a position adjacent the bottom of the lower partition;
closing, normally, said communication hole by the medical bag; pressing, from the outside, the medical bag for the separation and breakage of said separable weld for obtaining a mixture of the medical liquids between the partitions, which is followd by the opening of said communication hole, and;
thereafter, piercing said plug by said needle for discharging the medicines.

### EFFECTS OF THE INVENTION

In the present invention, the communication passage (hole) for the medical liquid is separately provided from the passageway for the vapor of medical liquid for sterilizing purpose, which allows size of the communication hole and size of the opening of the outlet port to be independently determined for obtaining respective desired values. Therefore, unlike the patent document No.1, a delicate adjustment of the small diameter value of the vent hole between the partition and outlet port is eliminated, which otherwise would be needed to obtain a free passage of the gas, on one hand and, on the other hand, to prevent a liquid flow from being generated. In addition, the communication hole is under a completely closed state under the non-open condition of the medical bag, which cooperates with the arrangement of the outlet port above the liquid level, thereby positively preventing such an erroneous operation from being occurred that an infusion of mere single liquid is practiced. The outlet port is, at its top end, opened to the lower partitioned chamber of the medical bag in the non-opened condition, and, therefore, under the suspended condition of a medical bag to an infusion stand, the upper end of the outlet port is located above the liquid level in the lower partition. Furthermore, the communication hole at the lower end is kept closed. Therefore, any discharge of the medical fluid is blocked even if an initiation of a infusion is attempted under the non-opened state of the medical bag. Therefore, an erroneous infusion operation, that only the medical liquid at one-sided partition is given, is prevented, which otherwise would occur when the opening operation of medical bag were neglected.

In a medical bag as a medical product, sterilization is essential after a filling process of medical liquids to the respective partitions. In the structure of the outlet port opened to the inner cavity of the medical bag according to the present invention, a vapor of the medical liquid as generated by the heating during the execution of the sterilizing process is introduced into the outlet port, thereby obtaining a positive sterilization effect under a wet heat condition. Finally, in the present invention, the outlet port of an increased inner diameter, itself, functions as a passageway for the vapor, resulting in an increased sterilization quality.

### BRIEF EXPLANATION OF ATTACHED DRAWINGS

Figure 1 is a schematic plan view of a multi-chamber container according to the present invention.
Figure 2 is a cross-sectional view taken along line II-II in Figure 1.
Figure 3 is a schematic plan view of a multi-chamber container according to the second embodiment of the present invention.
Figure 4 is a schematic plan view of a multi-chamber container according to the third embodiment of the present invention.
Figure 5 is a cross-sectional view taken along line V-V in Figure 4.
Figure 6 is a cross-sectional view taken along line VI-VI in Figure 4.
Figure 7 is a schematic plan view of a multi-chamber container according to the fourth embodiment of the present invention.
Figure 8 is a schematic plan view of a multi-chamber container according to the fifth embodiment of the present invention.
Figure 9 is a schematic plan view of a multi-chamber container according to the sixth embodiment of the present invention.
Figure 10 is a cross-sectional view taken along line X-X in Figure 9.

### EXPLANATION OF REFERENCE NUMERALS

10: Medical bag
12: Outlet Port
14: Strong Seal
14-1, 14-2: Expanded Portion of Strong Seal
18: Weak Seal (Separable Weld)
20, 22: Partition
24: Rubber Plug
26: Communication Hole
28: Valve Portion (Cooperating Member of the Invention)
30: Inner Passage of Outlet Port
31, 32: Reinforcing Part
34: Medical Liquid Passage
36, 38: Reinforcing Member
40, 42: Connector Portion
46, 48: Reinforcing Part
50, 52: Third, Fourth Partition
54: Medical Chamber
58: Vent Passage
62, 64: Communication Hole
74, 76, 78: Medical Chamber
80, 82, 84: Body Member for Medical Storage
86, 88, 90: Communication Hole

### BEST MODES FOR PRACTICING THE INVENTION

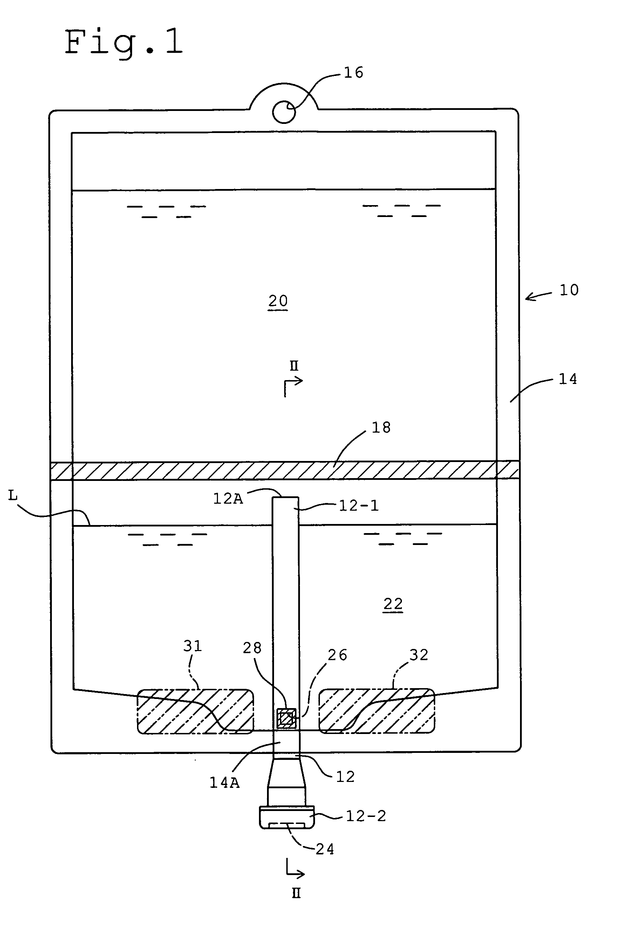

In Figure 1, a multi-chamber container includes a flat shaped medical bag 10 for storing therein with medical liquids and an outlet port mounted at the outer periphery of the medical bag 10. The medical bag 10 is formed from a soft synthetic resin film of a multi layer structure, such as a polyethylene film (flexible film of the present invention) of a thickness of a value such as 300 micron. Two cuts of synthetic resin film are provided, which are, at their outer peripheries, pressed at a temperature (about 130°C in case of polyethylene) sufficiently higher than its softening temperature, in order to create a strong seal (outer peripheral non-separable weld) 14, so that a bag of a substantial rectangular shape is obtained. Formed at the outer peripheral weld 14 is a suspension hole 16, by which the medical bag 10 is suspended and held by a infusion stand for practicing an infusion operation, such as intravenous feeding.

At a middle along the vertical direction of the medical bag, the upper and lower film layers constructing the medical bag are, along substantially entire width, welded at a temperature of a value about 120°C in case of polyethylene, which is somewhat lower than the temperature for obtaining the strong seal 14, so that a weak seal (separable weld) 18 is formed. The weak seal (partition wall) 18 divides the inner space of the medical bag 10 into upper and lower partitions 20 and 22, in which partitions medical liquids are respectively stored. As well known, prior to practicing the infusion operation, a portion of the partition 20 or 22, where the medical liquid is stored, is pressed from its outside, which causes the weak seal 18 is peeled and opened, so that the medical liquids in the partitions 20 and 22 are mixed.

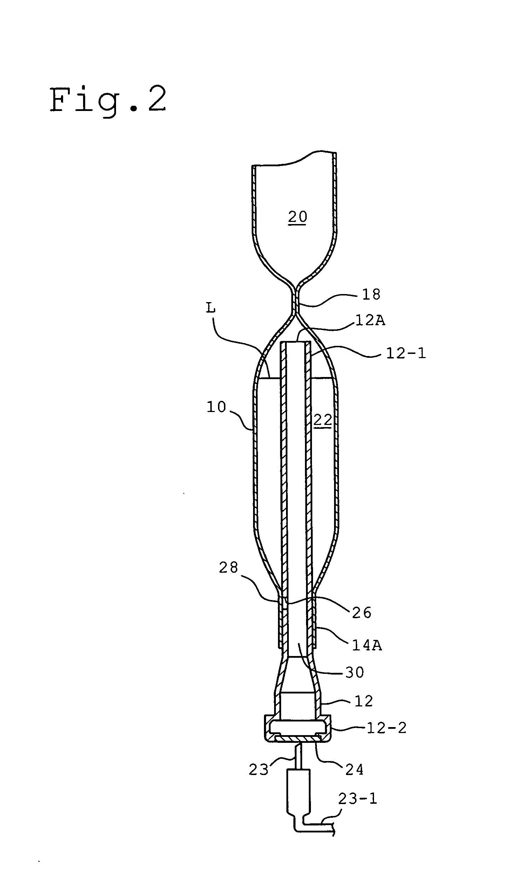

The outlet port 12 is made of a synthetic resin material of a value of wall thickness, which wall thickness is large enough to keep its tubular shape, and which material is preferably the same as that for the medical bag for obtaining a desired adherence capacity to the bag 12. In Figure 1, the outlet port 12 has a top end 12-1, opened to the lower partition 22 and a bottom end 12-2 fitted with a rubber plug (plug member) 24, which is pierced by a needle of an infusion set (not shown in Figure 1) for discharging medicines to a medical delivery pipe. Figure 2 illustrates a piercing type medical delivery device as an example of an infusion set, which includes a needle 23, from which a delivery pipe 23-1 is extended integrally. When the needle is pierced to the plug 24, medicines in the medical bag 10 is discharged to the medical delivery pipe 23-1. At the location of the outlet port 12, the top and bottom film cuts for constructing the medical bag 10 are, at their outer peripheries, non-separately welded to the opposed surface of the outlet port 12. The thus obtained strong seal portions are illustrated by the reference numeral 14A, for a distinction from the rest of strong weal illustrated by the reference numeral 14.

According to the present invention, the outlet port 12 extends in a manner that the top end 12-1 is located adjacent the weak seal 18, while its end opening 12A is opened the lower partition 22. As shown in Figures 1 and 2, L is a liquid level in the lower partition 22 as obtained when the medical bag 10 is held vertically by suspending the bag to an infusion stand by engaging the suspension hole 16 to the stand. As clearly shown, the opened end 12A of the outlet port 12 is opened to the bottom partition 22 at a location above the liquid level L. The outlet port 12 is formed with a communication hole 26 at a location adjacent the bottom of the lower partition but above the peripheral strong seal 14A. A valve part 28 (cooperating member), which normally closes the communication hole 26, is provided, which valve portion cooperates with the expansion of the medical bag as obtained when opening the medial bag. In the embodiment, the valve member 28 is constructed by a rectangular shaped area of the synthetic film constructing the medial bag and faced with the communication hole 26. The medical bag is, at the rectangular shaped area, separably welded to the outer surface of the outlet port 12. A temperature for the welding is of, for example, a value of about 125°C, which is somewhat larger than the welding temperature of 120°C for obtaining the weak seal 18 and is somewhat smaller than the welding temperature 130°C for obtaining the strong seal 14. This means that the peeling strength of the valve section 28 is larger than that of the weak seal 18. As a result, when a pressing of a location of the medical bag, where at the medical liquid is stored, a separation between the top and bottom films constructing the medical bag at the weak seal 18 occurs, first, in order to mix the medical liquids between the partitions 20 and 22. A further pressing of medically bag generates an increased peeling force, which causes the valve portion 28 to be separated, so that the mixed medical liquids is introduced into the outlet port 12 via the communication hole 26.

As a closure means for the communication hole 26, (a) the part of the synthetic resin film constructing the medical bag is separably welded as in the above embodiment. (b) In place of this, a separable film piece for closing the communication hole may be provided, which film piece is strongly (non-separably) welded to the opposed surface of the medical bag. Namely, the film piece usually closes the communication hole 26 and cooperates with the opening of the medical bag in a manner that the communication hole is opened. The structure is advantageous in easiness for controlling the degree of adherence (degree of strength of weak seal) of the film piece to the communication hole 26. (c) The outlet port may be provided with a locally thin walled portion, whereat a communication hole is to be formed. The locally thin walled portion is strongly welded to the opposed face of the medical bag by a welding means such as a point seal. An expanded deformation of the medial bag as generated upon the opening of the medical bag generates a force for breakage of the thin walled portion, thereby creating a communication hole. (d) A separate closure member may be provided, which is formed from a material such as a rubber and which is fitted to the communication hole, so that the latter is usually closed. The closure member is strongly welded to the opposite surface of the medical bag. An expanded deformation of the medial bag as generated upon the opening of the medical bag generates a force, which causes the closure member to be removed, thereby opening the communication hole. In any one of constructions of (a) to (d), a suitable adjustment of a difference in the opening strength of the communication hole 26 with respect to the weak seal (partition wall) 18 is essential in a manner that, after the completion of the opening of the weak seal, the opening of the communication hole is obtained when the further strengthened pressing is done.

In the operation of the embodiment in Figure 1, in order to open the weak seal 18 of the medical bag prior to the execution of an infusion operation, a portion of the medical bag contacting the stored liquid is pressed, resulting in a generation of a liquid pressure inside the bag, by which pressure the top and bottom films at the weak seal 18 is separated, resulting in a mixture of the medical liquids between the partitions 20 and 22. A further strengthened pressing of the medical bag is followed, which causes the medical bag is separated from the outlet port 12 at the valve part 28 to open the communication port 26, so that the medical liquid mixture is introduced into the inner passageway 30 of the inlet port 12 via the communication port 26. Therefore, a piercing of the rubber plug 24 by an infusion set (not shown) allows an infusion process to be initiated.

As explained above, the peeling of the weak seal 18 separating the upper and lower partitions 20 and 22 for mixing the medicines between the partitions is essential prior to practicing an infusion process. When this procedure were neglected, i.e., a piercing of the rubber plug by an infusion set were done under a non-opened condition of the medical bag suspended by a measure such as an infusion stand, the medical liquid only from the lower partition will be discharged, resulting in an erroneous infusion operation, which is avoided in the present invention. Namely, in the present invention, under a non-opened condition of the bag, wherein the weak seal 18 separates the top and bottom partitions 20 and 22, the top open end 12A of the outlet port 12 is located above the liquid level L in the lower partition 20 as shown in Figures 1 and 2. Furthermore, the medical bag is, at the area 28, welded to the opposed surface of the outlet port, i.e., the communication hole 26 is closed, so that the medicines in the lower partition 22 are prevented from being introduced into the inlet port 12, thereby preventing any occurrence of an erroneous infusion operation that the medicine only at the lower partition 22 is given. After the separation of the weak seal 18 for mixing of the medicines, a further pressing of the medical bag permits the valve member 28 to be separated from the outlet port 12 for opening of the communication hole 26, thereby allowing the mixed medicines to be introduced into the outlet port 12. Thus, a desired infusion operation can be initiated by piercing the rubber plug 24 by the infusion set.

As a medical product, a heat-sterilization should be done under a condition that the outlet port 12 is fitted to the medical bag 10 at its strong seal 14 and the medical liquids are filled under a sealed manner to the respective partitions 20 and 22 separated by the weak seal 18. According to the invention, the top end 12A of the outlet port 12 is opened to the inner space of the medical bag 10 at the lower partition 22. The space inside the outlet port 12 is filled by vapor of the medical liquid in the lower partition 22 as generated under a heating, resulting in an execution of the sterilizing process under a wet heat condition, thereby increasing a sterilization efficiency. Furthermore, a desired and stable sterilization performance is obtained, due to the increased inner diameter of the outlet port 12 as a passageway for the vapor.

As a partial modification of the first embodiment, reinforcing parts 31 and 32 formed as separable seals between the top and bottom film layers as shown in Figure 1 may be provided at both sides of the communication hole 26 in the outlet port 12. These reinforcing parts 31 and 32 are formed by separably welding top and bottom film layers constructing the medical bag. The reinforcing parts 31 and 32 function to block or reduce a force at the valve member 28 in a direction for separation of the valve member 28 as generated when the medical liquids stored in the medical bag is lightly pressed by a handling of the medical bag, such as a transportation, thereby preventing the communication hole 26 from being non-intentionally opened.

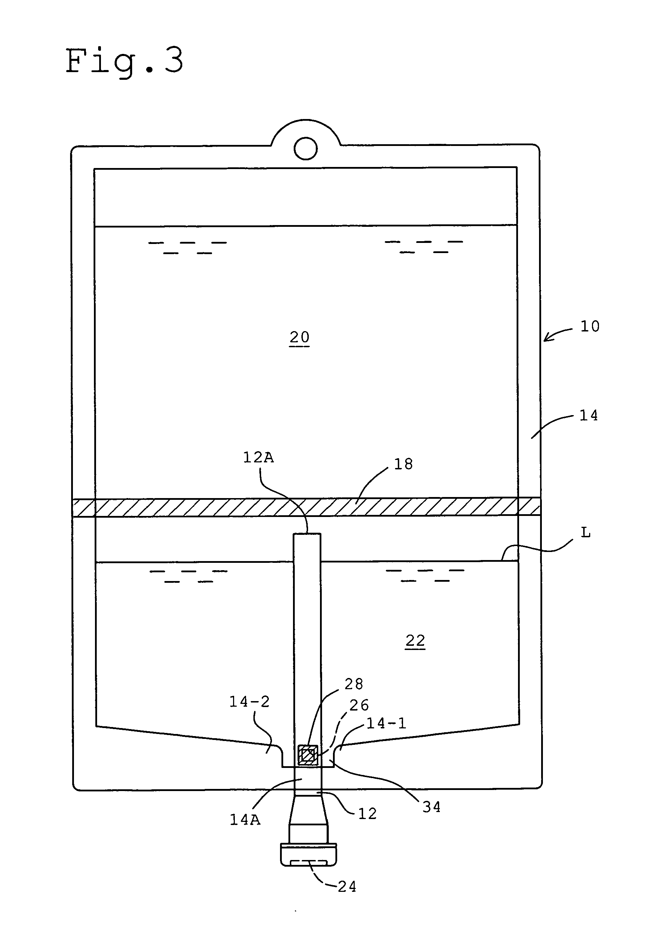

Figure 3 illustrates another embodiment of the present invention. In place of the separable welds 31 and 32 for strengthening the valve member 28 of the embodiment in Figure 1, a modified shape is provided for the strong seal 14 for a non-separable connection between the top and bottom layers at the outer periphery of the medical bag. Namely, the strong seal 14 is formed with a pair of expanded portions 14-1 and 14-2 extending from the sides of the strong seal 14 to locations adjacent the sides of the outlet port 12. The expanded portions 14-1 and 14-2 are located adjacent the sides of the outlet port 12 but are spaced from the outlet port 12 in a manner that flow passages to the communication hole 26 are left.

Since the portions 14-1 and 14-2 of the strong seal at the both sides of the communication hole 26 extend to the locations adjacent the outlet port 12 in the embodiment as shown in Figure 3, a relatively small force applied to the valve member 28 in the direction for opening the valve member is blocked or reduced, which force otherwise is generated even by a slight pressing of medical bag, which is inevitably generated during its handling, such as transportation. Thus, non-intentional opening of the valve member 26 is prevented.

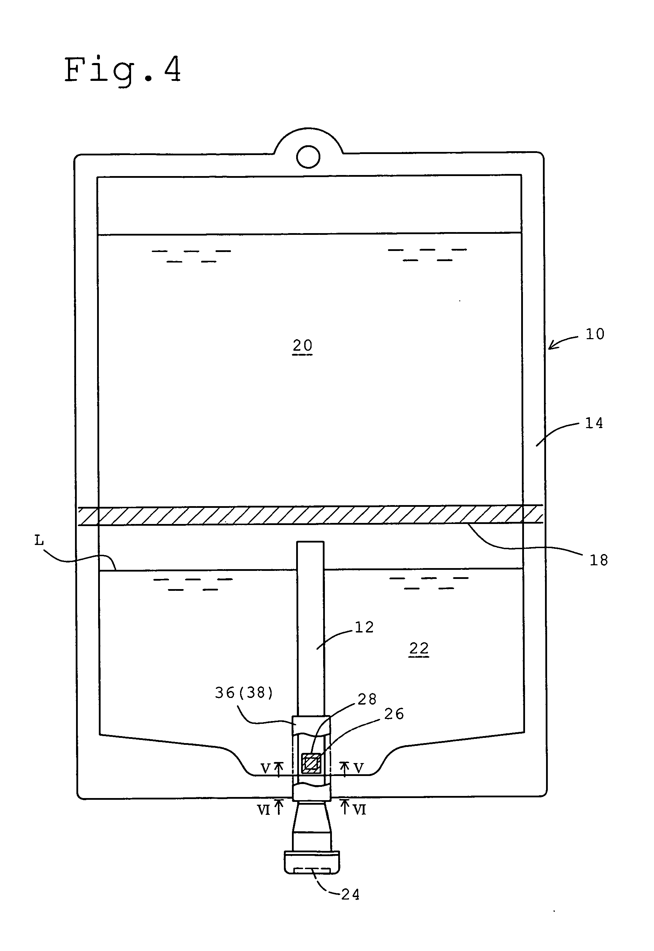

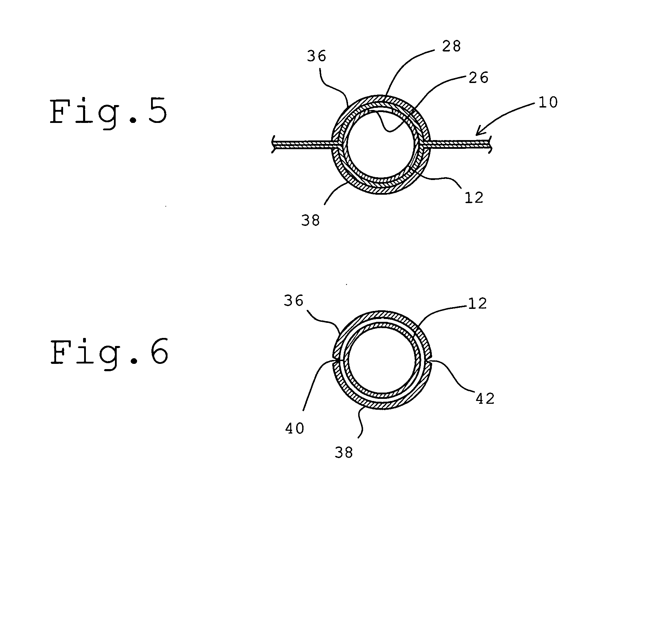

Figure 4 illustrates an embodiment having an outwardly attached reinforcing means. This outwardly attached reinforcing means includes a pair of reinforcing members 36 and 38 of semi-circular cross-sectional shape, which are connected to the medical bag 10 having the outlet port 12. The reinforcing members 36 and 38 have an inner diameter, which is slightly larger than the outer diameter of the outlet port 12. As s shown in Figure 5, the reinforcing members 36 and 38 at the top and bottom, respectively, are combined on the outlet port 12 via the medical bag 10 so as to form a substantial circular cross sectional shaped body by the members 36 and 38. Furthermore, a suitable means is provided at the outside of the medical bag 10 for obtaining a temporally integration of the reinforcing members 36 and 38 at the top and bottom, respectively. The means is realized, for example, by thin walled breakable portions 40 and 42, which integrally connects the top and bottom reinforcing members 36 and 38. As an alternative, such a temporally integration of the top and bottom reinforcing members 36 and 38 may be realized by a resilient engaging means based on a combination of groove and a projection. The integration means is, of course, not limited to the illustrated construction and may be realized by any suitable means.

In the embodiment in Figures 4 to 6, the reinforcing members 36 and 38 are arranged at outer surface of the outlet port 12 by way of the medical bag so as to function to isolate the valve member 28 from a separating force as generated when the bag is slightly pressed by an usual handling of a bag, such as transportation and to prevent the valve member from being accidentally opened. Namely, such a lighter separating force is not enough to break the thin walled connector portions 40 and 42, so that the reinforcing members 36 and 38 are kept at the combined state. Contrary to this, when the medical bag is opened, the medical bag is largely expanded, which causes an increased force to be generated, which force is large enough to break the connector portions 40 and 42, resulting in a separation of the valve member 28 from the outlet port 12 and in the opening of the communication hole 26.

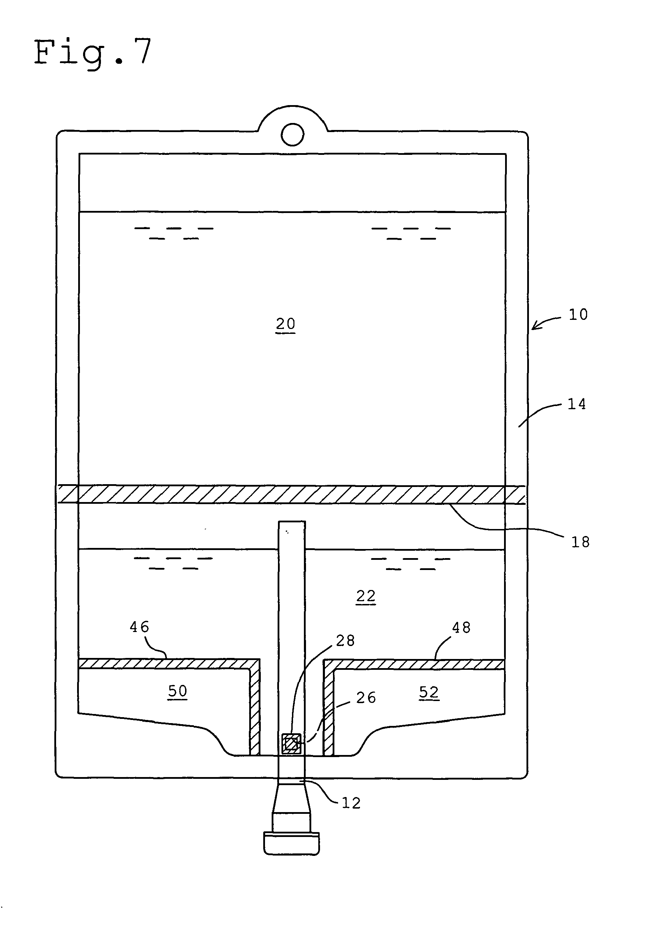

Figure 7 illustrates a further another embodiment, where reinforcing parts 46 and 48 as separable welds between the top and bottom layers of the medical bag are provided for creating additional partitions 50 and 52. The reinforcing parts 46 and 48, each of L-shaped, are arranges on the sides, respectively, of the outlet port 12. The reinforcing parts 46 and 48, each, has, with respect to the strong seal 14, a first end extending to its side portion of the strong seal 14 and a second end extending to its bottom portion, so that a third and fourth partitions 50 and 52 are created in the bag. The strength of the welds between top and bottom films is the same as that of the weak seal 18. In the partitions 50 and 52, respective medicines are stored. The reinforcing seals 46 and 48 have portions adjacent the sides of the communication hole 26, by which portions, a separation or reduction of a force peeling the valve member 28 as a section of the medical bag constructing the medical bag is obtained, as generated when the liquids in the medical bag is lightly pressed by a handling of the medical bag, such as its transportation, thereby preventing the communication hole 26 from being accidentally opened. Incidentally, the opening of the medical bag by a separation of the weak seal 18 causes the reinforcing seals 46 and 48 to be simultaneously opened, so that the medicines in the third and fourth chambers 50 and 52 are mixed to the medicines in the first and second partitions 20 and 22 and are introduced into the outlet port 12 via the communication hole 26.

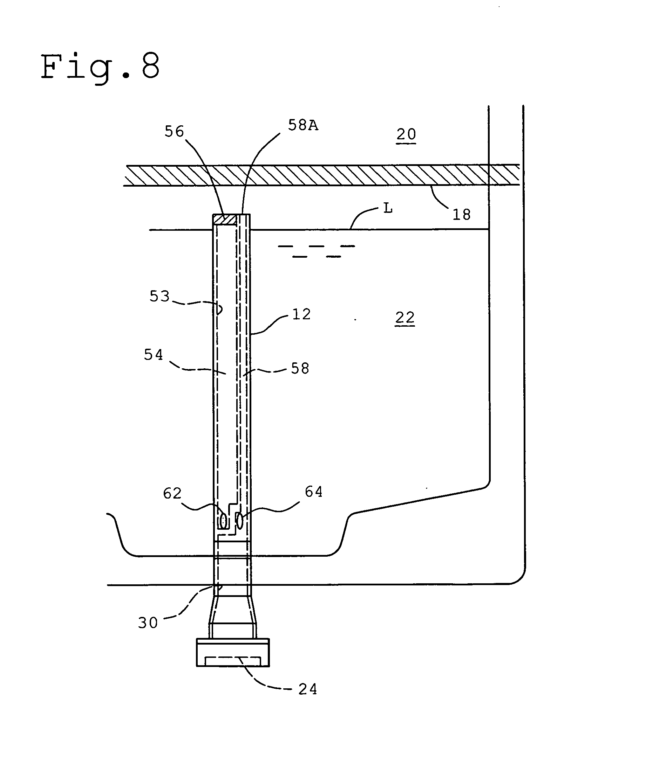

Figure 8 illustrates a further another embodiment of an outlet port 12, which is formed with a cavity 53 extending longitudinally. The cavity 53 has a first closed end adjacent the rubber plug 24 and a second open end fitted with a closure plug 56 in a manner that an additional medicine chamber 54 (an isolated chamber of the invention) is created. The outlet port 12 is formed with a vent passageway 58, which open at its bottom end adjacent the rubber plug 24 as well as at its top end adjacent the medical bag. The open top end 58A of the vent passage 58 is opened to the lower partition of the medical bag under a suspended condition at location above the liquid level L of the lower partition. The outlet port 12 is, at its outer periphery, further formed with a communication hole 61 opened to the medicine chamber 54 and a communication hole 64 opened to the vent passageway 58. The portions of the synthetic resin film constructing the medical bag opposite to the communication holes 62 and 64 are subjected to a separable welding to the outer surface of the outlet port 12, so that the communication holes 62 and 64 are normally under a closed state. Under the closed state of communication hole 62, a medicine(s) is able to be stored in the chamber 54. Under the closed state pf the communication hole 64, the medicines are prevented from being introduced into the outlet port 12. A valve member for closing and opening the communication hole 62 or 64 may be constructed in the same manner as the item (a) to (d) as explained with reference to the first embodiment.

In the operation of the embodiment in Figure 8, under the non-opened state of the medical bag, the communication holes 62 and 64 are closed by the opposite layer of medical bag, while the medical bag is under a suspended condition, which causes the top open end 58A of the vent passage 58 is located above the liquid level L in the lower partition 22. As a result, even an initiation of infusion were attempted by piercing the rubber plug 24 of the non-opened medical bag by an infusion set, any flow of the medicine does not occur, thereby preventing erroneous operation from being occurred. An expanded deformation of the medical bag as obtained when the medical bag is opened causes, at first, the weak seal 18 between the partitions 20 and 22 to be separated, resulting in mixing of the medicines therebetween. By a further strengthened pressing of the medical bag, the inner surface of the medical bag, which precedingly has closed the communication holes 62 and 64, are, now, dislaced from the outer surface of the outlet port 12, resulting in the opening of the holes 62 and 64. As a result, a small amount medicine(s) in the additional chamber 54 is combined with the mixed medical liquids from the partitions 20 and 22 and are in introduced into the inner passageway 30, which allows a desired infusion operation to be practiced by piercing the rubber plug 24.

In this embodiment, the vent passageway 58 connects the space inside the outlet port 12 to the space inside the medical bag, i.e., the lower partition 22. On the other hand, as a medical good, the multi-chamber container according to the invention must be subjected to a sterilization process under a condition that the outlet port 12 is mounted to the medical bag 10 at the strong seal 14 and the medical liquids are stored and sealed to the respective partitions 20 and 22. In this embodiment, the outlet port 12 is, at its opened top end 58A, opened to the inner chamber of the medical bag 10 (lower partition 22), so that the vapor as generated by the heat at the sterilizing process is introduced inwardly into the outlet port, thereby obtaining a positive sterilizing effect under a wet heat condition.

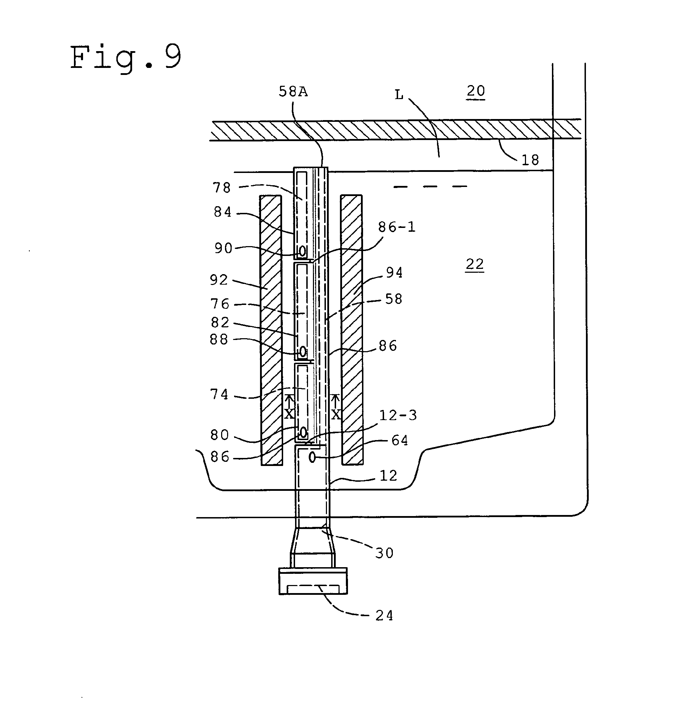

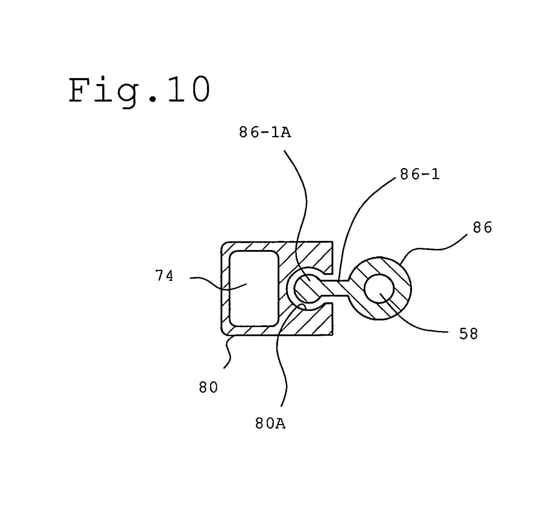

Figure 9 illustrates a further embodiment, which is similar to that in Figure 8 but different therefrom in that the outlet port 12 has a series of additional medical chambers 74, 76 and 78, which are arranged along the axial direction of the medical bag. The medical chambers 74, 76 and 78 are formed in body members 80, 82 and 84, respectively and each body member is formed of rectangular cross-sectional shape. A construction for mounting the body members 80, 82 and 84 to the outlet port 12 will, now, be explained. Namely, the outlet port 12 is formed with an extension part 86 of circular cross-sectional shape, which, in parallel with respect to the body members 80, 82 and 84, extends integrally form the end 12-3 adjacent the outermost body member 80. The extension part 86 is formed with a plate shape portion 86-1 extending, in cantilever fashion, integrally from a side thereof in a direction substantially parallel to the plane of the medical bag. The plate shape portion 86-1 has a free end 86-1A of an enlarged cross-sectional shape. The body members 80, 82 and 84 have sockets for snap engagement of the enlarged portion 86-1A for obtaining a fitting engagement of the body members 80, 82 and 84 to the outlet port. It should be noted that, in Figure 10, only the socket 80A for the body member 80 is shown. However, similar sockets are provided also for the body members 82 and 84. In place of such a fitting structure of the body members 80, 82 and 84 to the enlarged portion 86-1A, a permanent type integrated structure by means of welding, et al, may be employed. The body members 80, 82 and 84 are formed with communication holes 86, 88 and 90, respectively, which are opened to the medical chambers 74, 76 and 78, respectively. As similar to the embodiment in Figure 8, the outlet port 12 is formed with a vent passageway 58 and a communication hole 64 and the vent passageway 58 in the embodiment is formed in the integral extension part 86. A construction for obtaining a communication of the communication hole 64 to the vent passage 58 is also the same with that in Figure 8 in that the vent passageway 58 has a first end in communication with the rubber plug 24 via the inner passage 30 of the outlet port and a second end (top end) opened to the interior of the medical bag. The inner surface of the synthetic resin film constructing the medical bag opposite to the communication holes 64, 86, 88 and 90 are subjected to a separable welding to the outer surface of the outlet port 12. Under the closed state of the communication holes 64, 86, 88 and 90, the medical chambers 74, 76 and 78 store, therein, respective medicines. In addition, under the closed state of the communication hole 64, the medicines are prevented from being flown into the interior of the outlet port 12. As similar to the reinforcing parts 31 and 32 as explained with reference to Figure 1 in the first embodiment, reinforcing parts 92 and 94 are provided on sides of the outlet port 12 along thereto. These reinforcing parts 92 and 94 are illustrated by shaded areas on the sides of the communication holes 86, 88 and 90 to the medical chambers 74, 76 and 78, respectively and are constructed by welding the top and bottom synthetic resin films constructing the medical bag at a temperature, which is low enough to make the welds to be separable. As a result, a blockage or reduction of a force as generated when the medical liquids in the medical bag is lightly pressed during its handling such as a transportation to the portions (valve members) of the synthetic resin films closing the communication holes 64, 86, 88 and 90 is obtained and a non-intentional opening of the communication holes 64, 86, 88 and 90 is prevented. After the completion of the opening of the medical bag (mixing of the medical liquids between the partitions 20 and 22), the medical bag is further strongly pressed in a manner that the medical bag is expanded. In cooperation with such an expansion, the portions of the synthetic resin films facing the communication holes 64, 86, 88 and 90 are displaced and separated, resulting in opening of the communication holes 64, 86, 88 and 90. As a result, the medicines from the respective medical chambers 74, 76 and 78 are mixed with the mixed medicines from the partitions 20 and 22 and are flown into the interior of the outlet port 12 via the communication hole 64. Thus, an infusion process as intended can be initiated by piercing the rubber plug 24.

## Claims

1. A multi-chamber container comprising: a medical bag made of a flexible film; a separable weld, which connects under a separable manner the opposed surfaces of the medical bag in a manner that the inner space of the medical bag is separated into an upper and a lower partitions for storing medical liquids, respectively; an outlet port having a lower end located outside the medical bag and an upper end opened to the lower partition; a plug connected to the lower end of the outlet port, the plug being for piecing by a infusion set, said upper end of the outlet port opened to the lower partition being located above the level of the liquid in the lower partition under a non-opened condition of the medical bag, said outlet port being formed with a communication hole located at a position adjacent the bottom of the lower partition, and; a member normally closing said communication hole and cooperating with an expanded displacement of the medical bag as initiated when said weld is separated for opening the medical bag in a manner that the opening of the communication hole is succeeded.

2. A multi-chamber container as in claim 1, wherein said cooperating member is a portion of the medical bag opposite to the communication hole and said portion is separably welded to the opposed surface of the outlet port in a manner that said portion normally closes the communication hole.

3. A multi-chamber container as in claim 1, wherein said cooperating member is a film member fixedly connected to the surface of the medical bag opposite to the communication hole and said film member is separably welded to the opposed surface of the outlet port in a manner that said film member normally closes the communication hole.

4. A multi-chamber container as in claim 1, wherein said cooperating member is a valve member fixedly connected to the inner surface of the medical and said valve member is fitted to the communication hole in a manner that said valve member normally closes the communication hole.

5. A multi-chamber container as in claim 1, wherein said outlet port is, at its portion, constructed in a manner that it is locally fractured, the portion being fixedly connected to the opposed face of the medical bag and said communication hole is created due to the fracture of said portion as generated when the medical bag is opened.

6. A multi-chamber container as in any one of claims 1 to 5, wherein it further comprises additional separable welds connecting locally the opposite faces of the medical bag on both sides of the communication hole.

7. A multi-chamber container as in claim 6, wherein said additional separable welds form additional chambers for storing therein with medical liquids.

8. A multi-chamber container as in any one of claims 1 to 4, wherein it further comprises non-separable welds connecting the opposite faces of the medical bag on both sides of the communication hole, said non-separable welds being located adjacent to but spaced from the outlet and extending to the outer peripheral side portions of the medical bag.

9. A multi-chamber container as in any one of claims 1 to 8, wherein it further comprises a holder member arranged outside of the medical bag at a location opposite to the communication hole, said holder member cooperating with an expansion of the medical bag as generated when the latter is opened so that the holder member is separated from the medical bag.

10. A multi-chamber container as in any one of claims 1 to 9, wherein said outlet port is further formed with at least one normally closed, isolated chamber, which chamber is opened in cooperation with the opening of the medical bag.

11. A multi-chamber container as in claim 10, wherein said chamber is made separate from the outlet port.

12. A method for discharging medicines from a multi-chamber container to a medical delivery pipe connected to a needle, comprising the steps of:
providing, in the multi-chamber container, a medical bag made of a flexible film, a separable weld, which connects under a separable manner the opposed surfaces of the medical bag in a manner that the inner space of the medical bag is separated into an upper and a lower partitions for storing medical liquids, respectively, an outlet port having a lower end located outside the medical bag and an upper end opened to the lower partition, and; a plug connected to the lower end of the outlet port and
to be pierced by the needle connected to the medical delivery pipe;
locating said upper end of the outlet port in a manner that the upper end is opened to the lower partition at a position above the level of the liquid in the lower partition under a non-opened condition of the medical bag;
providing, in said outlet port, a communication hole located at a position adjacent the bottom of the lower partition;
closing, normally, said communication hole by the medical bag; pressing, from the outside, the medical bag for the separation and breakage of said separable weld for obtaining a mixture of the medical liquids between the partitions, which is followd by the opening of said communication hole, and;
thereafter, piercing said plug by said needle for discharging the medicines.
